# EUROPEAN PATENT APPLICATION

(11) **EP 4 775 119 A1**
(43) Date of publication of application: **15.07.2026**
(21) Application number: 24837976.0
(22) Date of filing: 06.11.2024

(54) **DEVICE FOR TREATING VERTEBRAL DEFORMITIES**

(71) Applicant: Burgos Flores, Jesús, 28042 Madrid (ES)
(72) Inventor: Burgos Flores, Jesús, 28042 Madrid (ES)
(74) Representative: Urizar Anasagasti, Jesus Maria
(86) International application number: PCT/ES2024/070692
(87) International publication number: WO 2026/099519

(57) **Abstract**

The present invention relates to a device for treating vertebral deformities, consisting of two rods (1), suitably curved to correct the deformity, which are attached by means of pedicle screws (2) at the level of the thoracic and lumbar vertebrae, comprising a plurality of segments (6, 3, 8), aligned and joined by a bushing (4), equipped with a transversal pin (7) that delimits the separation movement of the respective consecutive segments coupled thereto and, between two consecutive bushings (4), a bushing (5) sliding on the intermediate segment (3) on which it is mounted. A tightener (10) connects the first upper segment (6) forming each rod (1) to the last lower segment (8), passing through all the intermediate segments (3) and is provided with a tightening means that keeps all the segments aligned to form a rigid rod, and a loosening means that allows the separation between the segments and the displacement of the bushings (5) along the segment (3) on which they are mounted, so that the rod (1) can be lengthened to allow the patient's growth and the anteroposterior movement of the spine.

## Description

### Technical field

The invention falls within the sector of surgical instruments, devices and methods; in particular spinal stabilisers or positioners.

The object of the invention is a device for treating various vertebral deformities in children or young people the growth stage of whom is not yet complete. This device is particularly applicable in the correction of scoliosis and consists of a conventional vertebral attachment system using two rods which are attached to the thoracic and lumbar vertebrae by means of pedicle screws. This attachment system is characterised in that it allows two stages, one of total immobilisation of the treated vertebrae and another in which some mobility of the instrumented vertebral area is possible, in an anteroposterior direction. The change from one operating state to the other is determined and carried out by the medical specialist at their discretion. In addition, this device allows the patient's growth in the instrumented vertebral segment, once the device has acquired the mobility mentioned in the previous point.

### State of the art

The spine when viewed from the front or the rear is "straight". When it is not straight and has a curve to the sides, it presents a deformity called scoliosis. On radiographic study, the spine of a person with scoliosis looks more like a "C" or an "S" than a straight line, making the person's waist and/or shoulders appear uneven. In addition to the curves of the spine in scoliosis, the spine is rotated and this produces a "bulging of the ribs" (hump) on one side and a sinking of the ribs on the opposite side, producing an important aesthetic alteration that significantly affects patients and their families. This deformity of the spine can cause back and lower limb pain, especially in severe scoliosis which produce a degeneration of the affected spine and compression of the nerves leading from the vertebrae to the lower limbs.

When scoliosis occurs in adolescent patients aged ten years or older, it is called adolescent idiopathic scoliosis (AIS) and can be serious, leading to the aesthetic alterations described, as well as being associated with back pain and respiratory problems that can shorten the lives of these patients.

In general, curves greater than 25° in children require treatment with braces to prevent aggravation. Scoliosis of more than 35° require being treated with surgery to avoid the above mentioned consequences. Current techniques for the surgical correction of adolescent idiopathic scoliosis are focussed on correcting the spinal deformity and preventing its progression, and two surgical procedures have been developed to achieve these goals: The first consists of instrumental correction and fusion with attachment of the affected vertebral segment. The second, based on vertebral modulation, applies corrective forces to modulate the growth of the vertebral epiphyseal plates by means of vertebral implants.

Conventional surgery corrects scoliosis by leaving the spine fixed (without mobility), which has negative consequences for the unfixed mobile spine, because it degenerates exponentially over the years. In addition, the attachment of the thoracic spine produces a definitive respiratory restriction and, as metal implants are left in place for life, they also significantly increase the blood levels of these metals, with very severe consequences for these patients, who today have a life expectancy of more than 90-100 years. Poor quality of life and problems with pregnancy and childbirth have also been verified in patients treated with spinal fusion and instrumentation.

Surgical treatment of scoliosis in immature patients based on the modulation of vertebral growth is based on the Hueter-Volkmann Law, according to which compressive forces on the growth plates of immature patients cause slowing of physeal bone growth, and distraction forces increase bone growth. These forces can be applied to the scoliotic spine with the aim of restoring normal vertebral morphology. With this vertebral modulation technique, compressive forces are applied to decrease vertebral growth of the convexity and/or distraction forces are applied to increase vertebral growth of the concavity.

Two techniques based on vertebral modulation are Vertebral Body Tethering (VBT) and ApiFix, and both show severe short-term complications in almost 50% of cases, especially VBT. These techniques, as well as being not very predictable in terms of the final result, do not provide an acceptable aesthetic correction, and will also lead to disc degeneration in the non-instrumented mobile levels of the spine, as they leave residual curves in the instrumented area and will also lead to disc degeneration in the levels included in the instrumented area of the spine and in the instrumented levels having a minimal mobility and the discs are inclined in non-physiological positions, and will also be associated with reduced respiratory capacity and the serious complications already mentioned due to the high levels of metals in the blood.

Among sagittal plane deformities in children and adolescents, the most common is Scheuermann's kyphosis, consisting of a hyperkyphotic structural deformity of the thoracic or thoracolumbar spine, which, although generally benign in course, there is little information available about its natural history, in cases of severe disease it can progress into adulthood and cause significant deformity, disabling pain and respiratory distress. Conservative treatment using rehabilitation and orthoses lacks significant scientific evidence so surgical treatment must be considered in the presence of a progressive and painful kyphosis, neurological compromise, or unacceptable deformity. The current treatment is the same as for scoliosis in children and adolescents, the instrumental correction and subsequent fusion of the affected levels, and consequently these patients suffer the serious complications derived from this therapeutic method. To avoid them, the treatment we propose with this invention should be carried out in this group of patients, by correcting the deformity, allowing bone growth and vertebral movement during the time the instrumentation is maintained and after removing the instrumentation once bone growth is complete, with the aim of avoiding the short and long term consequences of vertebral fusion by maintaining metal implants in this group of patients for the rest of their lives.

In addition to this sagittal plane deformity, the model we propose in this invention, if it were to be used in all deformities in children and adolescents, could also be used in patients who have completed bone maturation a short time before, in whom this treatment would be carried out, to perform definitive vertebral attachment only at a few levels of the apex.

Among the aforementioned vertebral modulation techniques, the one developed in the following study stands out: Fusionless All-Pedicle Screws for Posterior Deformity Correction in AIS Immature Patients Permit the Restoration of Normal Vertebral Morphology and Removal of the Instrumentation Once Bone Maturity Is Reached, by Dr. Burgos et al (J. Clin. Med. 2023, 12, 2408), which reports a series of immature patients in whom a complete correction of the scoliotic deformity was performed by means of pedicle instrumentation, without fusion, which allowed normal vertebral morphology to be recovered after removal of the instrumentation, at the end of bone growth, achieving the correction of the aesthetic deformity, maintaining the mobility of the spine without progression of the scoliotic curve.

In summary, this technique of placing temporary metal implants in the vertebrae, which involves their subsequent removal when the patient's growth is complete, two or three years after the deformity has been corrected, makes it possible to maintain the correction obtained and normal vertebral mobility, without leaving metal implants in the spine.

In order to carry out this type of surgery to correct vertebral deformities, a multitude of vertebral implants have been developed, some of which allow some mobility of the rods with respect to the pedicle screws to which they are attached. Examples of such solutions can be found in documents US5593408 (A), US2021228242 (A1) or CN215839406 (U). Nevertheless, these rods do not meet the invention's objective of allowing the patient to grow, since this requires the rods attached to the vertebrae to lengthen, so that they can separate from each other to slightly vary the curvature of the spine and to lengthen and widen as occurs with the physiological growth of the patient. To meet this objective, the rods must be extensible, they must be able to lengthen with the patient's development and to lengthen and shorten in specific areas when flexing or extending this segment of the spine that has been instrumented, always in an anteroposterior direction, while maintaining the attachment in a lateral direction.

Also known in the prior art are US2019328424 (A1) and US2020197048 (A1), which disclose a segmented rod for aligning a spine, comprising a plurality of segments attached to a plurality of consecutive vertebrae, all of which are connected to an inner cable-tightener, which is attached to the first segment and runs to the lower end where it is wound up. In the second document, in contrast to the first, the segments have at least one channel and a pin facing this channel, so that the segments are coupled together without the possibility of rotation. These rods do not meet the objectives of the present invention as they are intended to be loosely attached with the segments separated, screwing each segment into consecutive vertebrae and, once that is done, to achieve the desired alignment of the spine, gradually actuating the tightener bringing the segments of the rod closer together as the cable is tightened; so that the segments gradually move closer together, the relative movement between the same being restricted until a solid rod assembly is formed, at which point the tightening stops. The solid rod then serves to maintain normal contoured vertebral alignment until vertebral growth is complete. At this point the cable is loosened and the patient is observed to ensure that the mature spine does not become misaligned and, if it does not, the device assembly is removed, leaving a normally functioning spine.

Continuing with the aforementioned studies, Dr. Burgos has come to the conclusion that the limitation of vertebral mobility and growth of the permanently instrumented vertebral segment has severe consequences for these patients, as it can cause cardiorespiratory deficits, early degeneration of adjacent vertebral segments and elevated blood levels of the metals that are used in the instrumentation. However, if mobility can be provided during the period of continued instrumentation until bone maturation, the possible consequences of immobilisation can be avoided.

It has also been found that complete instrumental immobilisation of the spine for a prolonged period of more than two years until the end of growth, in addition to causing limitation of spinal mobility after removal of the implants, prevents spinal growth during adolescence, which is a period of maximum body growth, and this loss of growth, in addition to reducing the height of these patients, would limit the size of the thoracic and abdominal cavities at the instrumented levels and consequently of the intrathoracic and intra-abdominal organs, and would produce a disharmony between the reduced growth of the intrathoracic and intra-abdominal organs with respect to the growth of the rest of the body, which could perhaps produce a lower blood or respiratory supply to the rest of the body than required or a greater demand on these organs.

For this reason, it would be advisable in this group of patients to use rods that initially allow the complete immobilisation of the instrumented spine or only by fixing the apex levels of the scoliosis of the treated vertebrae and, when the medical specialist considers it appropriate, to give anteroposterior mobility to the instrumented vertebral area in order to limit future vertebral mobility of the instrumented area as little as possible. In addition, these rods must have the ability to lengthen and allow for patient growth in all planes once the device has been removed.

### Explanation of the invention

The inventor has developed a method of surgical treatment that requires a device having the features of the present invention, this method comprising:
1. Implanting the pedicle screws on both sides of the spine, at the levels selected by the surgeon, and placing the two suitably curved rods thereon to correct the scoliosis in all planes. This surgical intervention is performed under general anaesthesia.
   The recommended procedure for this type of surgery is to first lower the convexity rod in a progressive manner and tightening all the screws successively and progressively to prevent these implants from being pulled out. Once said rod has been attached, the rod corresponding to the side of the concavity is placed, following the same process of successive and progressive tightening of the screws. Optionally, asymmetrical threaded bolts can be included in the concavity to prevent the central bolts from being pulled out.
2. Actuating means for partial release of said rods, in order to allow said rods to lengthen, so that when the pedicle screws are separated from each other, it provides flexing-extending mobility of the spine, while maintaining the lateral immobilisation of the rods, so that the subject can now move the spine in said direction and grow the instrumented portion of the spine without opposition from the rod. The operation that leads to this action could be performed under local anaesthesia, in two points of the spine, in which a device is manipulated in each rod that allows the vertebral rods to be released and, from that moment onwards, they can be lengthened. This actuation is performed when the complete recovery of the morphology of the vertebra or vertebrae at the apex of the curve is verified or when the surgeon considers it appropriate, which could generally be between six and twelve months after its implantation.
3. Finally, the rods and pedicle screws placed in the first phase are removed. This operation will be performed when the patient has completed bone growth (with Risser 5), generally between 2-4 years after implantation, and is intended to allow the patient to regain vertebral mobility and thus avoid the serious complications caused by leaving a vertebral segment without mobility and the metal implants inside the body. In other words, this avoids respiratory restriction, disc degeneration, rising metal levels in the blood, long-term poor quality of life and problems related to pregnancy and childbirth in these patients.

In order to achieve the proposed objectives, the invention proposes a device for treating various vertebral deformities in children and adolescents, having the features of claim 1.

Unlike other devices disclosed in the state of the art, which allow certain mobility at all times, from its implantation in the patient, the device of the invention must allow two well-defined situations: one of immobility, when it is implanted, and a second phase, which is initiated by manipulating the device, so that from then on it allows the lengthening of the rods and consequently the separation of the pedicle screws, so that the patient acquires certain mobility in an anteroposterior direction and the spine can grow, including in the instrumented area, avoiding a decrease in size in the thoracic and abdominal cavities and also a morphological alteration in the sagittal plane with possible repercussions on the organs located in the thorax and abdomen and altering the vertebral functionality in these segments.

In one embodiment, this device comprises two segmented rods, which in an initial position are solidified by means of internal tighteners connecting the upper segment to the lower segment via a series of intermediate segments. On the intermediate segments of which bushings are further arranged which form the surface on which the pedicle screws are attached, which, when released from the tension exerted by the corresponding tightener, allow a certain separation between all the pedicle screws and the lengthening of the rods, in order to allow the growth and mobility of the patient's spine in an anteroposterior direction.

In one embodiment, it is provided that the cross-section of the segments forming the rods and/or the inner cross-section of the bushings lining the intermediate segments is such as to prevent the latter from rotating with respect to the former. More specifically, a rod having a substantially square cross-section and rounded corners has been designed, which provides, among other advantages, the possibility that the segments can only move in the anteroposterior direction, thus avoiding the loss of complete scoliosis correction along the anteroposterior and axial planes. In addition, this square cross section of the rod allows the rods to be placed correctly on the screws, and that the position in which the rods are to be placed leaves the surgeon no doubts as to which side of the rod should be towards the back or to either side, or resting on the base of the pedicle screws.

In a preferred embodiment, it is provided that the rods are shaped prior to surgical implantation in a way that is customised for each patient, with the appropriate incurvations, thereby greatly facilitating the implantation of the rods and achieving complete three-dimensional correction of scoliosis with minimal gestures. It would be ideal to have a customised conformation system for each patient, for example, a system based on a machine learning model, obtained from the training of multiple surgical cases carried out in the past.

In at least one embodiment, it is provided that this device can be manipulated back to a position in which the vertebral rods become solid and attached again, disabling their lengthening again, at least temporarily.

In one embodiment, it is also provided that the mobility of the instrumented spine is progressively less from the most proximal vertebral level to the most caudal level, as in a normal spine.

With these rods their use is not limited to the treatment of idiopathic scoliosis, they can also be used for treating scoliotic and sagittal plane deformities of any aetiology in children and adolescents, taking into account the important advantages of their use by allowing vertebral growth and mobility without leaving metallic implants inside the body at the end of the treatment.

Similarly, vertebral deformities in the sagittal plane, such as Scheuermann's disease, congenital kyphosis and other aetiologies could be treated with this device, maintaining the correction of the affected vertebral segments obtained intraoperatively by immobilising the anterior bending of the affected vertebral segment in the anterior plane, but allowing lateral extension and bends.

This novel technique avoids major complications of spinal fusions because a large number of metal implants are kept inside the body, which can lead to carcinogenesis, immune deficiencies, genetic alterations and high levels of metals in the blood. Similarly, conventional spinal fusion causes a decrease in respiratory capacity, triggers early and exponential degeneration of the vertebral levels not included in the instrumentation, aesthetic alterations and possible complications derived from thoracoabdominal underdevelopment by reducing the size of the thoracic and abdominal cavities with respect to the rest of the body.

### Description of the drawings

As a complement to the present description, and for the purpose of helping to make the features of the invention more readily understandable, said description is accompanied by a set of drawings which, by way of illustration and not limitation, represent the following:
- Figures 1 and 2 show schematically one of the two rods (1) of the device of the invention in its operative position initially immobilising the spine and once it has been released and lengthened due to the patient's growth.

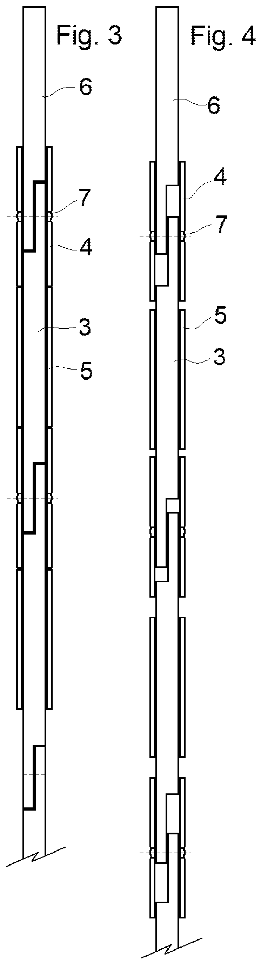

- Figures 3-4 respectively show cross-sectional views from lateral planes of one of the rods (1) that make up this device, with its segments (6, 3) together, forming a solid rod, which corresponds to the fixing position in the first stage of the treatment and a position in which these segments are separated from each other, while the bushings (5) are separated from the bushings (4), during the second stage of the treatment in which the patient has grown and/or acquired some mobility in the anteroposterior direction.

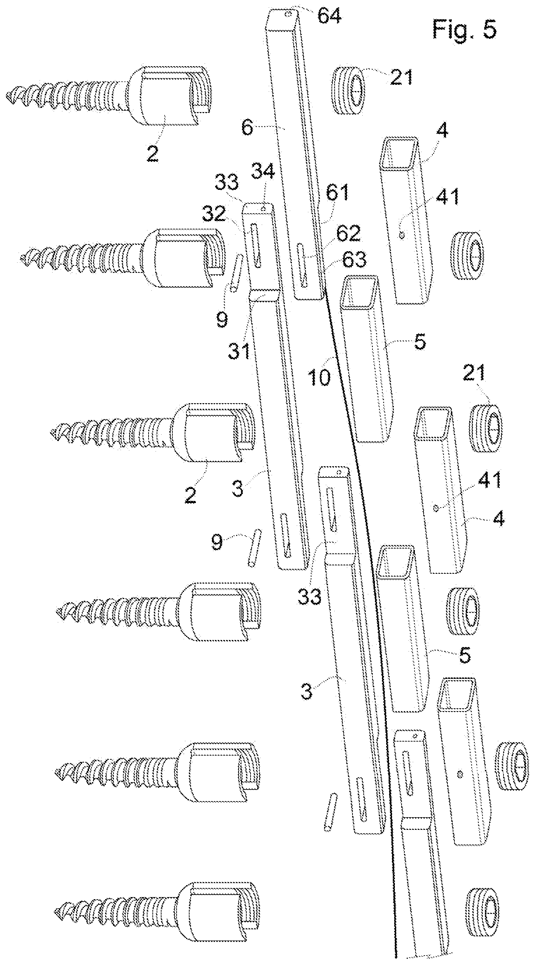

- Figure 5 shows a view with all the elements that make up this device (except the lower segment (8)), disassembled and in a deployed position.

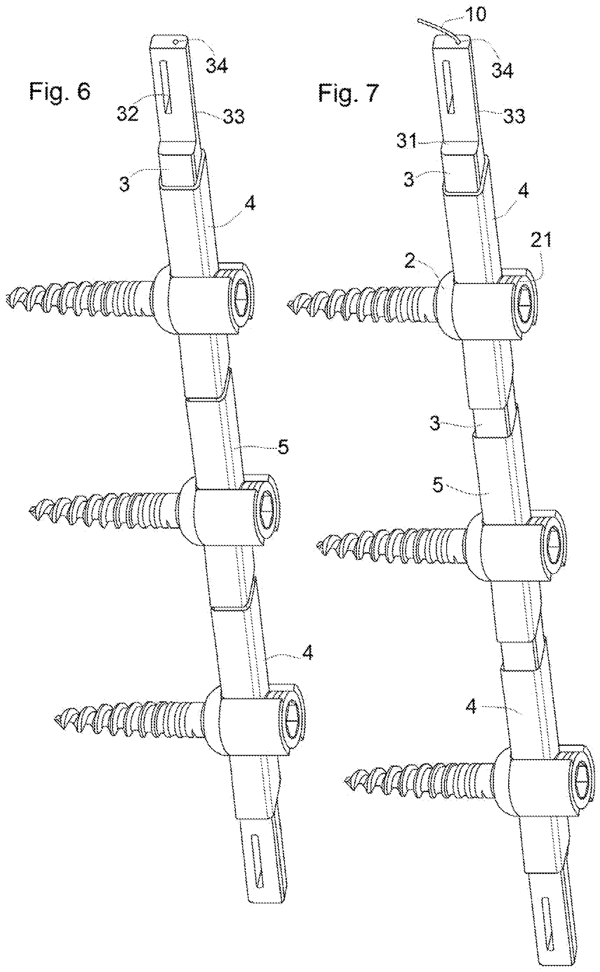

- Figures 6-7 show two perspective views of a portion of a rod in the initial position and once the means allowing the separation between the pedicle screws (2) attached thereto has been released.

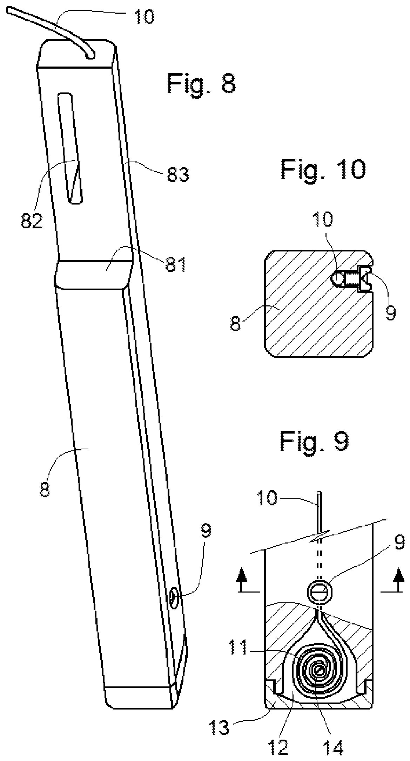

- Figure 8 shows a perspective view of the segment (8) in which the rod (1) ends at the bottom.
- Figure 9 show a cross-sectional view of one embodiment of the lower end of the segment (8).
- Figure 10 shows a cross section of said segment (8), through the area of the screw (9) that forms the locking/unlocking means of the tightener (10).
- Figures 11-14 represent different cross sections that the segments can have and the bushings (4, 5) mounted or attached thereto.
- Figures 16-18 respectively show an intermediate area of a rod in which two consecutive segments (3) are joined together for immobilising the vertebra(e) of the apex of the scoliotic curve.

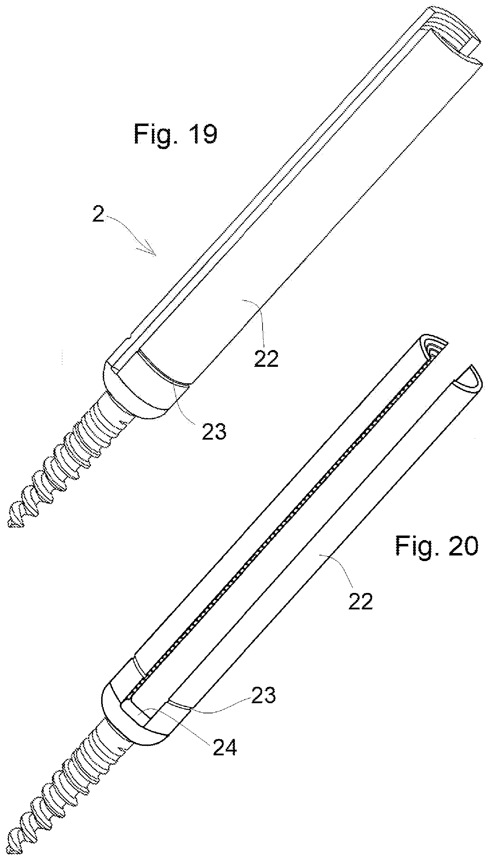

- Figures 19-20 show two perspective views of one type of pedicle screw (2) that is used in this device.

### Embodiment of the invention

The device of the present invention has been designedin order to carry out the proposed method for treating scoliosis, device which is made up of two rods (1), suitably curved in the lateral plane to provide the normal curve that the dorsal and lumbar spine have in this plane, while in the frontal-posterior plane the rod is straight or with minimal curvature towards the side opposite to the curvature presenting the scoliosis. These rods are implanted into the pedicle screws (2), which were previously placed at the thoracic and lumbar levels of the spine as planned by the surgeon. This correction is achieved by previously shaping each of the rods (1) with the appropriate curvature in the anteroposterior direction, so that when they are attached to the pedicle screws (2), each of them pushes the vertebrae in the opposite direction to the one in which it is laterally inclined, so that the spine, after attaching this device, returns to its normal position without lateral incurvations or rotations and with the physiological sagittal incurvations through the modulation of growth, which occurs in the vertebral epiphyseal plates of children.

The device of the invention must allow two situations: a first one, in which the attachment of the spine in the affected area is unmovable, and a second one, which allows the movement of a wide segment of the thoracolumbar spine, which is triggered by manipulating this device, which causes the rods (1) to be able to lengthen and shorten and, consequently, the pedicle screws (2) to separate from each other and come closer together again to adapt to the patient's growth and for the patient to acquire the possibility of movement, only in the anteroposterior direction.

Each of said rods (1) of this device comprises a series of segments (6, 3, 8), aligned and joined together by means of a means that allows an initial position in which the set of segments forms a solid rod, and a position in which said segments can be slightly separated. All segments have means at least at one end that allow an initial position in which the set of segments (6, 3, 8) forms a solid rod, and a position in which said segments can be separated from each other. These means consist of a lateral recess (31, 61, 81), starting from a central plane in an anteroposterior direction, which on the opposite side define an appendix (33, 63, 83), which is equipped with a longitudinal mounting hole (32, 62, 82), which, by facing and overlapping two consecutive segments with each other in this area, the facing mounting holes form a transverse window with respect to the spine, through which a pin (9) joins the two overlapping segments with a bushing (4), allowing longitudinal displacement of said segments as far as a tightener (10) allows. The first upper segment (6) at its upper end and the lower segment (8) at its lower end have no such recess so that the rod ends with a uniform cross-section at both ends.

All segments are connected by at least one tightener (10) which is attached to the first upper segment (6) forming each rod (1), and is immobilised in the last lower segment (8), passing through all intermediate segments (3). This tightener (10) is provided with a tightening means that keeps all the segments aligned to form a solid rod, and a loosening means that allows a separation between consecutive segments. These means will be explained in more detail below.

A bushing (4) is mounted on the joint between two consecutive segments (6, 3) (3, 3) (3, 8), which internally has a configuration homologous to said segments, so that the segments can be moved longitudinally along the corresponding bushing (4). Each bushing (4) has a transverse pin (7) that connects the two segments that meet at this joint through the corresponding mounting holes (32, 62, 82) of said two segments, so that when the tightener (10) allows, a movement of the segments away from and back closer together is enabled, in order to allow the distension of the rod (1) in an anteroposterior plane of the spine.

A bushing (5) is also mounted on the intermediate segments (3), which can slide along the corresponding segment (3). When the rod is in the compressed position, each bushing (5) is inserted between two consecutive bushings (4), in contact or in close proximity to each other, so that the set of bushings (4) connecting two consecutive segments and the bushings (5), sliding on the intermediate segments (3), form a sleeve over the set of intermediate segments (3), to which a series of intermediate pedicle screws (2) are attached, with the exception of one or two pedicle screws (2) that are attached to the upper segment (6) and another pedicle screw (2) that is attached to the lower segment (8), once all the aforementioned screws have been previously attached to the successive vertebrae to be treated. When the tightener (10) connecting all the segments (6, 3, 8) is released, the sleeve formed by the set of bushings (5 and 4), acquires the ability for lengthening and shortening to the original position, due to the possibility of separation between two consecutive segments and displacement of the bushings (5) along the segment (3) in which they are mounted.

It is intended that the sum of the number of bushings (4) connecting two consecutive segments and the number of bushings (5) sliding on the intermediate segments (3) is equal to or greater than the number of vertebrae in the length of said segments, when the rod (1) is in a compressed and solid position; in this way, no two pedicle screws (2) can coincide on the same bushing (4) or (5) and consequently, once the tightener (10) is released, all screws can be slightly separated from each other.

Taking into account that two half bushings (4) and a sliding bushing (5) are mounted in each segment (3) and that at the joint between two segments the pin (7) can be moved from end to end inside the mounting holes of two consecutive segments, the permissible separation between two consecutive bushings (4, 5) or (5, 4) is similar to the length of said mounting holes. In a limit embodiment, which would be that provided for implantation in young people with a Risser 0 or 1 and who are therefore expected to grow a lot, the lengthening degree of the rod would be approximately 20 %, which would in most cases allow the implant to be kept for 4-6 years, without affecting the patient's growth.

Moreover, it is provided that the cross section of the intermediate segments (3) and/or the inner cross section of the bushings (4, 5) is such that it does not allow the rotation of the bushings with respect to the intermediate segments (3), thus avoiding the rotation of the bushings on the pedicle screws and consequently the loss of correction of the scoliotic rotation, as well as its lateral movement, so the spine, once the tightener (10) has been released, can only move in an anteroposterior direction, i.e. the direction in which the pedicle screws (2) can be slightly separated or moved closer together, and consequently the vertebrae thereby attached.

The tightener (10) consists of a cable attached to the first upper segment (6) forming each rod (1), which passes through all the intermediate segments (3), through a longitudinal hole (34) therein, and emerges at the end of the lower segment (8), or is wound into a cavity (12) at the lower end of said lower segment (8). The tightener (10) may be made of plastic, aramid, steel, or any other material with high tensile strength so that it can have a minimum cross section.

The tightening means that keeps all the segments (6, 3, 8) aligned, forming a solid rod, and the loosening means that allows a separation between two consecutive segments, consists of a screw (9), accessible from the face towards the patient's back of the lower segment (8), which presses the tightener (10) in an initial position in which the rod is solid, or releases the same to allow the longitudinal displacement between the segments and the displacement of the sliding bushings (5) on the intermediate segments (3) and consequently the separation between the pedicle screws (2) with growth, or with the movements of the patient's spine in the anteroposterior direction.

In one embodiment, the free end of the tightener (10) is wound onto a spool (11) located in a cavity in the lower portion of the lower segment (8).

In one embodiment, the spool (11) on which the tightener (10) is wound is provided with an actuation means (14) which allows it to be rotated and the tightener cable (10) to be wound therein, should it be necessary to re-immobilise and stiffen the rod (1).

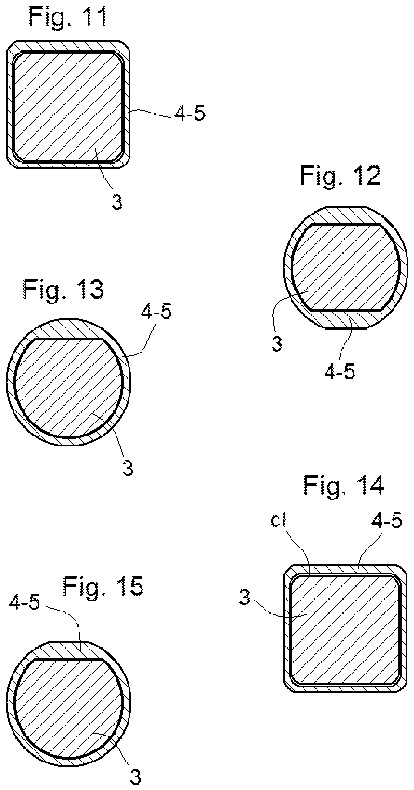

As stated above, the cross-section of the intermediate segments (3) and/or the inner cross-section of the bushings (4, 5) is such that the bushings cannot rotate with respect to the intermediate segments (3). In a preferred embodiment, the segments (6, 3, 8) and the bushings (4, 5), attached or mounted thereon, have a quadrangular cross-section with rounded corners. (See Figure 11, for example).

The cross-section of the segments (6, 3, 8) may also be a circular segment the chord of which is perpendicular to the anteroposterior plane of the spine, while the bushings (4, 5) attached or mounted thereon have a cross-section internally homologous to that of the said segments and externally a circular cross-section, or circular with one or two cuts according to chords perpendicular to the anteroposterior plane of the spine. (See Figures 12, 13, 15)

Figure 14 shows an embodiment in which, between the segments (6, 3, 8) and the bushings (4, 5), attached or mounted thereon, there is a slight clearance (cl) allowing at any time a slight movement in an anteroposterior direction of the spine attached with rods (1) formed with segments and bushings with said clearance.

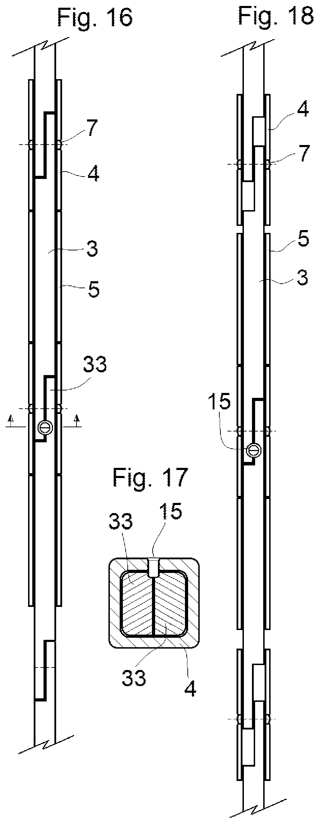

Figures 16-18 show an intermediate area of a rod in which two consecutive segments (3) are joined together in order to prevent the mobility of a specific area of the spine because it is impossible for said segments to separate, even though the tightener (10) has been released. The specific area to be immobilised is normally that which coincides with the vertebra or vertebrae at the apex of the scoliotic curve, as these are the vertebrae most affected and it is possible that in certain patients or under certain circumstances the surgeon may prefer to keep them immobilised.

The attachment between two segments (3) is carried out by means of a screw (15) which is threaded into the bushing (4) from the rear face thereof and into the opposite faces of the appendages (33) of said segments (3). This screw (15) can be removed to also provide mobility to this area, when the full recovery of the morphology of the vertebra(s) at the apex of the curve is verified.

In an alternative embodiment the two segments (3) joined together are replaced by a single segment of a length suitable to span the entire vertebra(s) at the apex of the scoliotic curve.

In an alternative embodiment, the length of the mounting holes (32) progressively increases in length from the segments (6, 3) located at the thoracic vertebral level to the segments (3, 8) coinciding with the most caudal lumbar level, in order to provide greater mobility of the instrumented spine, as occurs in a normal spine.

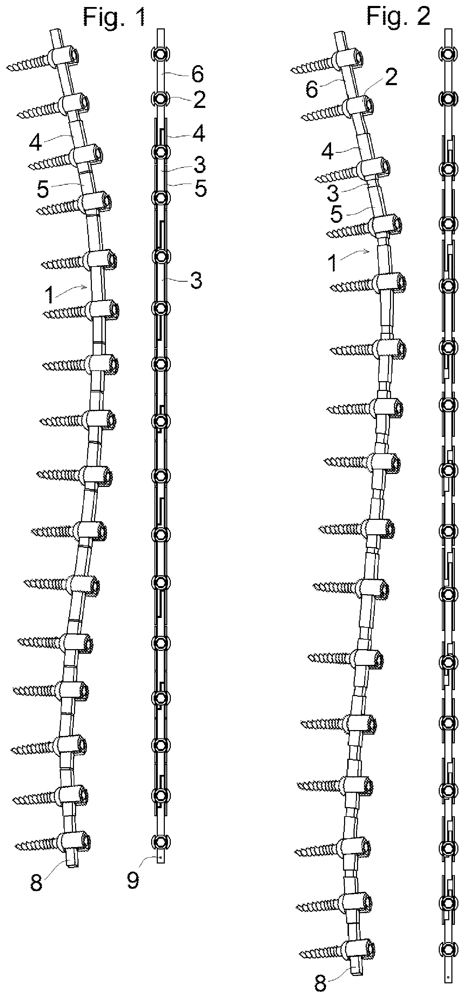

In a preferred embodiment, the rods (1) are shaped prior to implantation, depending on the radiological characteristics of the observed deformity and the patient's measurements, so that when implanted on the screws, following a well-established sequence, the complete three-dimensional correction of the deformity is achieved. This shaping is carried out with the rod compressed and solid, due to the tightening between the different segments (6, 3, 8) carried out by the tightener (10), as shown in Figure 1.

Likewise, the rods (1) are preferably manufactured or shaped with a certain number of segments, so that they have a length close to the length of the patient's spine section to be attached, at the time of implantation.

To attach this device, the first step is to shape the rods (1). This shaping operation can be carried out in the factory, by the specialist surgeon before starting the operation, or by means of an equipment equipped with a shaping mechanism according to certain physical characteristics of the patient, preferably on the basis of an artificial intelligence programme. In any case, the rods are mounted when they form a solid body by the tightening carried out by the tightener (10) between the different segments (6, 3, 8) forming the same, as can be seen in Figure 1.

Both the rods (1) and the screws (2) are made of titanium, chrome vanadium or any other materials that are suitable for use in this sector. In general, ferromagnetic metals are not used in order to allow a patient carrying such implants to undergo magnetic resonance imaging.

In a preferred embodiment, screws (2) having long bells (22) are used, even longer on the convexity than on the concavity of the scoliotic curve, in order to facilitate the lowering of the rod on this side, which has a greater curvature, so that the rotation and consequently the rib hump can be corrected.

The screws (2) have a threaded stud for insertion into the vertebra to be attached, from which two bells (22) start, facing each other and threaded internally, in which a nut (21) tightens and immobilises the corresponding rod (1), once it occupies the lower seat (24) that separates the two bells at the bottom. The screws (2) of the long bells (22) have a breakage strip (23) in a lower area, close to the area where the nut (21) is located when the rod (2) is tightened against the seat (24). In general, the seat (24) is configured according to the rod (1); nevertheless, in some embodiments, this seat and screw (2) can be conventional, with the insertion of an intermediate bushing or gasket to fill the spaces between this gap and the rod used. In one embodiment, the separation between the lampshades (22) is slightly wider than the width of the rod (1), in order to facilitate sliding down the rod at the time of its fitting; in this case, said separation and the seat (24) are in accordance with the configuration of the rod.

It is also stated for the appropriate purposes that the materials, shape, size and arrangement of the elements described may be modified, as long as this does not imply an alteration of the essential features of the invention that are claimed below:

## Claims

1. A device for treating vertebral deformities, consisting of two rods (1), suitably curved to correct three-dimensionally and completely the deformity of the spine, which are attached by means of pedicle screws (2) in the thoracic and lumbar vertebrae, wherein each of said rods (1) **comprises:**
- a plurality of segments (6, 3, 8), aligned and joined together by means of a means allowing an initial position in which the set of segments forms a solid rod, and a position in which said segments can be separated from each other;
- a tightener (10) connecting the first upper segment (6) forming each rod (1) to the last lower segment (8), passing through all the intermediate segments (3), which is provided with a tightening means that keeps all the segments aligned forming a solid rod, and a loosening means that allows a separation between consecutive segments;
- a series of bushings (4), each of which is mounted on the joint between two consecutive segments (6, 3) (3, 3) (3, 8), and equipped with a transverse pin (7) that delimits the movement of separation of the respective consecutive segments coupled thereto, allowing the loosening of the rod (1) in the anteroposterior plane of the spine; and
- a series of bushings (5), sliding on the intermediate segments (3), mounted between two consecutive bushings (4) and close to the same when the rod is in the compressed position, the set of bushings (4) connecting two consecutive segments and the bushings (5) sliding on the intermediate segments forming a sleeve on the set of intermediate segments (3), to which the pedicle screws (2) are attached, with the exception of one or two that are attached to the upper segment (6) and another one that is attached to the lower segment (8), once the pedicle screws (2) have been attached to the successive vertebrae to be treated, which sleeve, when the tightener (10) connecting all the segments (6, 3, 8) is released, acquires the possibility of lengthening and shortening to the original position, due to the possibility of separation between two consecutive segments and of displacement of the bushings (5) along the corresponding segment (3) on which they are mounted.

2. The device according to claim 1, wherein the cross-section of the intermediate segments (3) and/or the inner cross-section of the bushings (4, 5) is such that the bushings cannot rotate with respect to the intermediate segments (3).

3. The device, according to any of the preceding claims, wherein the sum of the number of bushings (4) connecting two consecutive segments and the number of bushings (5) sliding on the intermediate segments (3) is equal to or greater than the number of vertebrae present in the length of said segments, when the rod (1) is in a compressed and solid position.

4. The device, according to any of the preceding claims, wherein the means allowing an initial position in which the set of segments (6, 3, 8) form a solid rod, and a position in which said segments can be separated from each other comprises at the ends of the segments an area in which two consecutive segments overlap, facing a lateral recess (31, 61, 81) starting from a central plane in an anteroposterior direction, with an appendage (33, 63, 83) defined on the opposite side, which is equipped with a longitudinal mounting hole (32, 62, 82), which when facing and overlapping two consecutive segments with each other in this area, the mounting holes are facing each other, defining a transverse window with respect to the spine, through which a pin (9) joins the two overlapping segments with a bushing (4), allowing the longitudinal displacement of said segments as far as the tightener (10) allows.

5. The device, according to any of the preceding claims, wherein the tightener (10) consists of a cable attached to the first upper segment (6) forming each rod (1), which passes through all the intermediate segments (6), through a longitudinal hole (34) therein, and emerges at the end of the lower segment (8), or is wound into a cavity (12) at the lower end of said lower segment (8).

6. The device, according to claim 5, wherein the tightening means that keeps all segments (6, 3, 8) aligned to form a solid rod, and the loosening means that allows a separation between two consecutive segments, consists of a screw (9), accessible from the face towards the patient's back of the lower segment (8), which presses the tightener (10) in an initial position in which the rod is solid, or releases the same to allow the longitudinal displacement between the segments and the displacement of the bushings (5) sliding on the intermediate segments (3) and consequently the separation between the pedicle screws (2), with growth, or with the movements of the patient in the anteroposterior direction.

7. The device, according to any of claims 5-6, wherein the free end of the tightener (10) is wound on a spool (11) located in a cavity in the lower portion of the lower segment (8).

8. The device, according to claim 7, wherein the spool (11) on which the tightener (10) is wound is provided with an actuation means (14) for it to be rotated and the tightener cable (10) to be wound therein, should it be necessary to re-immobilise and stiffen the rod (1).

9. The device, according to claim 2, wherein the segments (6, 3, 8) and the bushings (4, 5) attached or mounted thereon have a quadrangular cross-section with rounded corners.

10. The device, according to claim 2, wherein the segments (6, 3, 8) have a cross section according to a circular segment the chord of which is perpendicular to the anteroposterior plane of the spine, while the bushings (4, 5) attached or mounted thereon have internally a cross section homologous to that of said segments and externally a circular cross section, or circular with one or two cuts according to chords perpendicular to the anteroposterior plane of the spine

11. The device, according to any of the preceding claims, wherein, between the segments (6, 3, 8) and the bushings (4, 5) attached or mounted thereon, there is a slight clearance (cl) allowing at any time a slight movement in an anteroposterior direction of the spine attached with rods (1) formed with segments and bushings with said clearance.

12. The device, according to any of the preceding claims, wherein, in an intermediate area of the rods (1), two consecutive segments (3) are joined, disabling the mobility of that specific area of the spine, once the tightener (10) has been released.

13. The device, according to claim 12, wherein the attachment between two segments (3) is carried out by means of a screw (15) which is threaded into the bushing (4) from the rear face thereof and into the opposite faces of the appendages (33) of said segments (3).

14. The device, according to any of claims 1-11, wherein in an intermediate area of the rods (1) one of the intermediate segments (3) is approximately twice as long as the remaining ones.

15. The device, according to any of the preceding claims, wherein the length of the mounting holes (32) progressively increases in length from the segments (6, 3) located at the thoracic vertebral level to the segments (3, 8) coinciding with the most caudal lumbar level.

16. The device, according to claim 1, wherein the shape of the rods (1) is customised prior to implantation, based on certain physical characteristics of the patient.

17. The device, according to claim 1, wherein the rods (1) are manufactured or shaped with a certain number of segments, so that they have a length close to the length of the patient's spine section to be attached, at the time of implantation.

18. A method for treating vertebral deformities, comprising:
- implanting two suitably curved rods (1) to correct the existing vertebral deformity three-dimensionally and completely, which are attached by means of pedicle screws (2) in the thoracic and lumbar vertebrae,
- operating means for unlocking said rods (1) to allow their lengthening, so that the pedicle screws acquire a certain mobility in the anteroposterior direction, while continuing to immobilise the spine in the lateral direction, and so that the rods (1) can be lengthened in accordance with the patient's growth.
- removing the rods and pedicle screws placed in the first phase.

19. The method according to claim 18, wherein the actuation on the unlocking means of the rods (1) attached to the spine is carried out between 6-18 months, or when the surgeon considers it appropriate after implantation.

20. The method according to claims 18-19, wherein the removal of the rods and pedicle screws placed in the first phase is carried out between two and six years, or when the surgeon considers it appropriate, after implantation.
